# EUROPÄISCHE PATENTSCHRIFT

(11) **EP 2 780 025 B1**
(45) Veröffentlichungstag und Bekanntmachung des Hinweises auf die Patenterteilung: **16.12.2015**
(21) Anmeldenummer: 12775513.0
(22) Anmeldetag: 24.10.2012
(51) Int. Cl.: A61K 36/73, A61P 29/00

(54) **VERWENDUNG VON EXTRAKTEN AUS FILIPENDULA ZUR BEHANDLUNG UND PROPHYLAXE VON CHRONISCHEN SCHMERZZUSTÄNDEN**
USE OF EXTRACTS FROM FILIPENDULA FOR THE TREATMENT AND PROPHYLAXIS OF CHRONIC PAIN CONDITIONS
UTILISATION D'EXTRAITS DE FILIPENDULE POUR LE TRAITEMENT ET LA PROPHYLAXIE D'ÉTATS DOULOUREUX CHRONIQUES

(30) Priorität: 28.10.2011 DE 102011085413
(43) Veröffentlichungstag der Anmeldung: 24.09.2014
(73) Patentinhaber: Dr. Willmar Schwabe GmbH & Co. KG, 76227 Karlsruhe (DE)
(72) Erfinder: KOCH, Egon, 76229 Karlsruhe (DE); MÜSCH, Werner, 76227 Karlsruhe (DE); NÖLDNER, Michael, 76139 Karlsruhe (DE); SCHÖTZ, Karl, 76448 Durmersheim (DE)
(74) Vertreter: Adam, Holger
(86) Internationale Anmeldenummer: PCT/EP2012/071031
(87) Internationale Veröffentlichungsnummer: WO 2013/060714

(56) Entgegenhaltungen:
- EP-A1- 0 507 035
- WO-A1-2004/062682
- WO-A2-03/004000
- WO-A2-2008/008474
- US-A1- 2007 134 195
- US-A1- 2007 286 899
- BALÃ ZS BLAZICS ET AL: "LCâ MS Qualitative Analysis and Simultaneous Determination of Six Filipendula Salicylates with Two Standards", CHROMATOGRAPHIA ; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, Bd. 71, Nr. 1, 14. April 2010 (2010-04-14) , Seiten 61-67, XP019810117, ISSN: 1612-1112

## Beschreibung

Die vorliegende Erfindung betrifft die Verwendung von Extrakten aus Filipendula zur Behandlung oder Prophylaxe von chronischen Schmerzzuständen ohne erkennbare organische Ursachen.

Chronische Schmerzen sind mehr oder weniger starke Schmerzen, die eine oder mehrere Körperregionen betreffen und über mehr als 3 Monate anhalten. An der Entstehung von chronischen Schmerzen können unterschiedliche Prozesse beteiligt sein (z.B. Trauma, Nervenschädigungen, vorhergehende Erkrankungen oder psychische Faktoren), weshalb die Symptomatik chronischer Schmerzen auch verschiedenen Indikationsgebieten zuzuordnen ist (z. B. ICD-10 F45 "Somatoforme Störungen", G43 "Migräne", G44 "Sonstige Kopfschmerzsyndrome", R51 "Kopfschmerz", R52 "Schmerz, anderenorts nicht klassifiziert", M79.7 "Fibromyalgie" R20.2 "Parästhesie der Haut", K58 "Reizdarmsyndrom", N94.3 "Prämenstruelle Beschwerden"). Dabei sind die chronischen Schmerzzustände, die von diesem Patent berührt werden nur solche, bei denen keine akuten organischen Gründe für die Schmerzen nachgewiesen werden können.

Chronischer Schmerz grenzt sich deutlich vom akuten Schmerz ab, da akuter Schmerz eine klare physiologische Funktion, nämlich die Vermeidung weiterer schädlicher Einflüsse auf das entsprechende Gewebe oder den gesamten Organismus hat. Akuter Schmerz wird durch einen akuten Reiz (z.B. Temperatur, Druck, Verletzung, Infektion) ausgelöst, ist meistens lokal auf die Stelle der Einwirkung begrenzt, wird als "scharf" empfunden und führt zu einer Schutz- und Vermeidungsreaktion, die weiteren schädlichen Einfluss verhindern soll. Wenn die Beeinträchtigung vorüber ist, vergeht auch der Schmerz.

Bei chronischem Schmerz ist diese physiologische Funktion nicht vorhanden. Der Schmerz ist nicht mit einer akuten Gefahr einer physiologischen Beeinträchtigung verbunden. Häufig werden chronische Schmerzen auch weniger lokal gerichtet und eher als "dumpf" und tiefer im Gewebe liegend empfunden. Chronische Schmerzen gehen nicht zurück. Zur typischen Symptomatik chronischer Schmerzen zählt die Hyperalgesie, bei der die Schmerzen beim gleichen Reiz wesentlich stärker empfunden werden, als dies normalerweise der Fall wäre. Ein anderes Beispiel ist die Allodynie, bei der ein Reiz als schmerzhaft empfunden wird, der normalerweise keinen Schmerz auslöst.

Auch mechanistisch sind akute und chronische Schmerzen gut unterscheidbar. Ein akuter Schmerz entsteht durch die Reizung nozizeptiver Nervenenden (entweder direkt oder über Rezeptoren) und wird als Aktionspotential über Rückenmark und Gehirn verarbeitet. Dabei treten klare Aktivierungen von Nerven auf, die danach wieder in den Ausgangszustand zurückgehen. Wenn die periphere Reizauslösung verhindert wird, kann auch der Schmerz vermieden werden. Zusätzlich können Substanzen im zentralen Nervensystem die Schmerzverarbeitung beeinflussen und so analgetische Empfindungen auslösen.

Bei chronischen Schmerzen treten andere Reize des Nervensystems auf. Es wird heute davon ausgegangen, dass Fehlregulationen des Nervensystems z.B. eine Enthemmung des NMDA-Rezeptors und fehlerhafte Verarbeitung der Schmerzprozesse im Gehirn an chronischen Schmerzen beteiligt sind (Villman and Becker 2007; Neuroscientist; 13 (6); 594-615 / Woolf and Salter 2000, Science; 288; 1765-1768). Dabei sind an der Schmerzempfindung auch lern- und psychosoziale Prozesse beteiligt. Das führt als komplexes und multifaktorielles System zu einer dauerhaften Schmerzwahrnehmung, für die in vielen Fällen keine körperliche Ursache vorhanden ist. Da nicht nur "typische" Prozesse der Schmerzverarbeitung beteiligt sind, haben weder analgetische Substanzen die peripher die Reizleitung verhindern (z.B. Lidokain) noch viele zentral aktive Analgetika einen längerfristigen therapeutischen Nutzen (Mello and Dickenson 2008; British Journal of Anaesthesia; 101 (1); 8-16 / Hucho and Levine 2007; Neuron; 55; 365-376). Dazu kommt häufig ein problematisches Nebenwirkungsspektrum solcher Substanzen (z.B. Opioide).

Dagegen werden für viele chronische Schmerzerkrankungen im Einzelfall psychoaktive Medikamente wie z.B. Pregabalin, Ketamin oder Milnacipran eingesetzt, deren Wirkungen aber begrenzt sind (Lawson 2008; Drug Discovery Today; 13 (7-8); 333-340 / , Häuser et al 2009; JAMA; 301 (2); 198-209 / Sud et al. 2008; European Journal of Pharmacology; 588; 217-231). Es existieren deshalb keine allgemein wirksamen Standardbehandlungen für chronische Schmerzen. Zusätzlich sind die angesprochenen psychoaktiven Substanzen auf Grund ihrer neurologischen Nebenwirkungen häufig kontrainduziert.

Bei vielen Erkrankungen mit chronischen Schmerzen ohne körperliche Ursachen wird Stress als wichtiger auslösender oder begleitender Parameter diskutiert. Es ist bekannt, dass andauernder Stress häufig mit der Symptomatik chronischer Schmerzen einhergeht und diese auch im Tierversuch auslösen kann (Dina et al. 2009; Neuroscience; 160; 501-507 / Imbe et al 2006; Frontiers in Bioscience; 11; 2179-2192 / Vidal and Jacob 1986; Annals New York academy of sciences; 73-81).

Aufgabe der vorliegenden Erfindung ist es somit ein Mittel bereitzustellen, das wirksam zur Behandlung von chronischen Schmerzzuständen ohne erkennbare organische Ursache verwendet werden kann und weitgehend frei von Nebenwirkungen ist.

Diese Aufgabe wird erfindungsgemäß gelöst durch die Verwendung von Extrakten aus Filipendula-Arten, bevorzugt aus Filipendula ulmaria zur Therapie und Prophylaxe von chronischen Schmerzzuständen ohne erkennbare organische Ursachen, besonders von chronischen Schmerzen, die durch somatoforme Störungen verursacht sind, und von Vulvodynie sowie von Abdomen-, Kiefergelenk, sonstige Gelenk-, Extremitäten-, Nacken-, Schulter-, Rücken-, Lenden-, Becken- oder Wirbelsäulenschmerzen nichtrheumatischen Ursprungs, sowie von chronischen Schmerzen, die durch Reizdarmsyndrom oder durch prämenstruelle Beschwerden, durch Fibromyalgie oder durch Parästhesie der Haut oder Migräne, Cluster-Kopfschmerz, chronische paroxysmale Hemikranie, vasomotorischen Kopfschmerz, Spannungskopfschmerz oder chronischen posttraumatischen Kopfschmerz induziert sind. Bevorzugt werden hierbei die oberirdischen Pflanzenteile (Kraut) eingesetzt.

Filipendula wird in die Familie der Rosaceae eingeordnet und umfasst erfindungsgemäß die folgenden Arten: F. ulmaria, F. angustiloba, F. digitata, F. formosa, F. glaberrima, F. kamtschatica, F. kiraishiensis, F. multijuga, F. occidentalis, F. palmata, F. purpurea, F. rufinervis, F. rubra, F. vestita und F. vulgaris (syn. F. hexapetala). Eine bevorzugte Filipendula Art ist Filipendula ulmaria. Eine weitere bevorzugte Filipendula Art ist Filipendula vulgaris. Eine weitere bevorzugte Filipendula Art ist Filipendula purpurea. Das Verbreitungsgebiet der bevorzugten Pflanze Filipendula ulmaria erstreckt sich über die nördlichen Regionen Europas, Amerikas und Asiens. Die volksmedizinischen Anwendungen sind im Bereich der Erkältungskrankheiten und der rheumatoiden Erkrankungen (als Erkrankungen unter Beteiligung entzündlicher Prozesse) zu finden, wobei als Arzneidroge sowohl Blüten, als auch die getrockneten oberirdischen Teile der blühenden Pflanze verwendet werden (Kraut). Filipendula ulmaria enthält Salizylsäurederivate und wird mit der Entwicklung von Aspirin in Verbindung gebracht. Aspirin als Salizylsäurederivat gehört in die Gruppe der nichtsteroidalen Antirheumatika (NSAIDs), deren gemeinsames Wirkprinzip die Hemmung der Cyclooxygenasen 1 und 2 (COX 1 und 2) darstellt. Durch die Hemmung der COX-Enzyme wird die Entzündungskaskade gehemmt, da die Cyclooxygenasen die entzündungsvermittelden Prostaglandine aus Arachidonsäure herstellen. Die Prostaglandine sind neben der eigentlichen Entzündung auch an der Vermittlung entzündungsbedingter Schmerzen beteiligt, weshalb NSAIRs auch Anwendung bei akutem, entzündungsvermitteltem Schmerz finden (Brune 2004; Rheumatology; 43 (Suppl.1; i16-i20) / Wallace 2007; British Journal of Pharmacology; 152; 421-428).

Überraschenderweise wurde jetzt allerdings festgestellt, dass Extrakte aus Filipendula in Tiermodellen für nichtentzündliche chronische Schmerzzustände wirksam sind, was auf deren therapeutische Verwendbarkeit bei chronischen Schmerzzuständen ohne erkennbare organische Ursachen schließen lässt. Eine derartige Wirkung ist für Filipendula-Extrakte bisher nicht beschrieben und war aufgrund der bisher für Filipendula bekannten pharmakologischen und klinischen Effekte nicht zu erwarten.

Zur Herstellung der im Rahmen der vorliegenden Erfindung verwendeten Extrakte aus Filipendula wird das getrocknete und gemahlene Pflanzenmaterial mit einem organischen Lösungsmittel oder Wasser oder einem Gemisch aus einem oder mehreren organischen Lösungsmitteln und/oder Wasser bei einer Temperatur zwischen 10 °C und 100 °C extrahiert. Das ausextrahierte Pflanzenmaterial wird von der Extraktlösung, z. B. durch Filtration, abgetrennt und ggf. erneut mit einem Lösungsmittel entsprechend dem ersten Schritt extrahiert und ebenfalls von der Extraktlösung abgetrennt. Die so gewonnenen Extraktlösungen werden vereinigt, eingedampft und getrocknet.

Bevorzugte organische Lösungsmittel für die Extraktion sind Alkohole oder Ketone, vorzugsweise Ethanol oder Aceton und deren Gemische mit Wasser. Besonders bevorzugt sind Gemische aus Ethanol und Wasser im Gewichtsverhältnis von 20/80 bis 80/20 (20 Gew.-% bis 80 Gew.-%), vorzugsweise 50/50 bis 70/30 (50 Gew.-% bis 70 Gew.-%), sowie Wasser. Als Extraktionsverfahren kommen z.B. Mazeration oder Perkolation in Frage (vgl. Europäisches Arzneibuch, Ausgabe 6.0). Die Trocknung kann durch an sich bekannte Verfahren wie z. B. Gefriertrocknung oder Trocknung im Vakuum bei Raumtemperatur oder erhöhte Temperatur erfolgen. Zur Anreicherung von ausgewählten Inhaltsstoffen können weitere Konzentrierungsschritte durchgeführt werden wie z. B. flüssig-flüssig-Verteilung mit z. B. 1-Butanol/Wasser oder Ethylacetat/Wasser, Adsorption-Desorption an Ionenaustauscher, Sephadex LH20, Diaion HP20 und andere Harze oder chromatographische Abtrennungen über RP18, Kieselgel, etc..

In einer bevorzugten Ausführungsform wird ein Teil gemahlenes Kraut (oberirdische Teile) der gewünschten Filipendula-Art wird mit fünf bis zehn Teilen 50 Gew.-% bis 70 Gew.-% Ethanol 1/2 Std. bis 3 Std. bei 50 °C bis 60 °C gerührt. Das ausextrahierte Pflanzenmaterial wird von der Extraktlösung durch Filtration abgetrennt und erneut mit fünf bis zehn Teilen 50 Gew.-% bis 70 Gew.-% Ethanol 1/2 Std. bis 3 Std. bei 50 °C bis 60 °C gerührt und abfiltriert. Die vereinigten Filtrate aus den beiden Extraktionsschritten werden im Vakuum bei 40 °C bis 60 °C vom Ethanol befreit und gefriergetrocknet und/oder im Vakuum bei 40 °C bis 60 °C im Trockenschrank getrocknet.

Die Extrakte können in Form von Tropfen, Pulvern, Granulaten, Tabletten, Dragees oder Kapseln vorzugsweise oral verabreicht werden. Möglich ist aber auch eine parenterale Anwendung in Form einer Injektionslösung oder eine topische Anwendung in Form von Cremes, Salben, Suppositorien, Pflastern oder ähnliche Zubereitungen.

Zur Herstellung von Tabletten wird der Extrakt mit geeigneten pharmazeutisch bzw. lebensmittelrechtlich akzeptablen Hilfstoffen wie z. B. Laktose, Cellulose, Siliciumdioxid, Croscarmellose und Magnesiumstearat gemischt und zu Tabletten gepresst, die gegebenenfalls mit einem geeigneten Überzug z. B. aus Hydroxymethylpropylcellulose, Polyethylenglykol, Farbstoffen (z. B. Titandioxid, Eisenoxid) und Talkum versehen werden.

Die Extrakte können auch, ggf. unter Zusatz von pharmazeutisch bzw. lebensmittelrechtlich akzeptablen Hilfsstoffen wie z. B. Stabilisatoren, Füllmittel etc., in Kapseln abgefüllt werden.

Die Dosierung erfolgt dabei so, dass pro Tag 5 bis 2000 mg, bevorzugt 10 bis 1000 mg, besonders bevorzugt 60 bis 600 mg Extrakt zugeführt werden.

Die Extrakte sowie die daraus hergestellten Produkte können sowohl als Arzneimittel als auch als Lebensmittel zum Einsatz kommen. Unter Lebensmittel sind hierbei insbesondere diätetische Lebensmittel, Nahrungsergänzungsmittel sowie "medical food" und "dietary supplements" zu verstehen.

Die Wirksamkeit der Extrakte aus Filipendula wird durch die nachstehend beschriebenen Versuche belegt.

### Pharmakologische Untersuchungen

### Schwimmstress induzierte Hyperalgesie:

Die Schmerzempfindlichkeit von Ratten wird auf einer heißen Platte getestet. Das Lecken des Fußes oder Springen wird als Schmerzreaktion gewertet. Die Zeit bis zum Auftreten dieser Reaktion wird gestoppt und als Maßstab für das Schmerzempfinden verwendet. Schwimmstress über drei aufeinanderfolgende Tage (jeweils 30 min) löst ein deutliches Absinken der Schmerzschwelle (Hyperalgesie) aus. Dieser Effekt kann durch Behandlung mit Extrakt aus Filipendula ulmaria gemäß Beispiel 1 oder Ketamin 5 mg/kg (Referenzsubstanz) rückgängig gemacht werden.

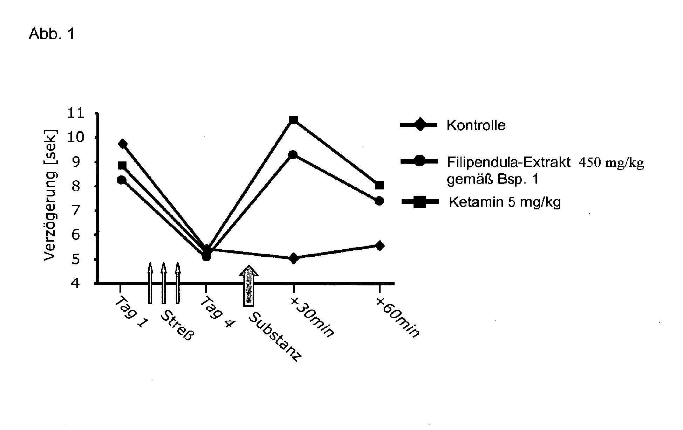

*Abb. 1* *zeigt den Einfluss von Extrakt aus Filipendula ulmaria gemäß Beispiel 1 auf die durch Schwimmstress induzierte Hyperalgesie. Angegeben sind die Basalwerie der Reaktionszeiten auf der Hot Plate an verschiedenen Tagen (1 und 4), sowie die Zeiten 30 und 60 min nach der p.o.-Applikation von Lösemittelkontrolle, Extrakt oder Referenzsubstanz an Tag 4 (Pfeil).*

### Glucocorticoid induzierte Hyperalgesie:

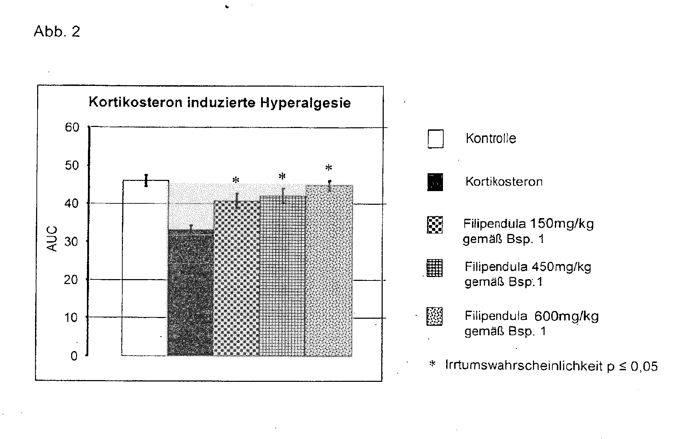

Physiologisch werden Stressreaktionen zum großen Teil über die Freisetzung von Glucocorticoiden vermittelt (Kolber et al. 2008; Stress; 11(5); 321-338). Durch Verabreichung von Corticosteron im Trinkwasser über einen Zeitraum von 14 Tage wird in Ratten eine chronische Stresssituation simuliert, die eine Hyperalgesie auslöst. Diese Hyperalgesie wird über eine Apparatur zur Bestimmung der Schmerzschwelle bei Ausübung eines zunehmenden Drucks auf eine Hinterpfote gemessen. Dabei wird die Zeit bis zum Wegziehen der Pfote als Schmerzreaktion gewertet. Es werden an Tag 15 über einen Zeitraum von 8 Stunden 6 Messungen durchgeführt (0, 1/2, 1, 2, 4, 8 Std.) und daraus die "Area under the Curve" (AUC) berechnet. Wie aus Abb.2 hervorgeht, hemmt der Extrakt aus Filipendula ulmaria gemäß Beispiel 1 in allen untersuchten Dosierungen die durch Corticosteron induzierte Hyperalgesie.

*Abb. 2* *zeigt den Einfluss von Extrakt aus Filipendula ulmaria auf die Corticosteron induzierte Hyperalgesie. Angegeben ist die "Area under the Curve" der Zeitverlaufsmesskurven. *: Irrtumswahrscheinlichkeit p*≤*0,05*

### Beispiel 1: Extrakt aus Filipendula ulmaria

600 g fein gemahlenes Kraut (oberirdische Teile) von Filipendula ulmaria werden zwei mal mit je 4200 g 60 Gew.-% Ethanol jeweils 1 Std. bei 60 °C gerührt, anschließend die Suspension über eine Fritte P4 abgesaugt, die vereinigten Filtrate im Vakuum bei 60 °C vom Ethanol befreit, der verbliebene wässrige Rückstand eingefroren und lyophilisiert. Der erhaltene Feststoff wird im Vakuum bei 40 °C über P₂O₅ und KOH getrocknet: 146.2 g (24.4%) Trockenextrakt.

### Beispiel 2: Tabletten

Ein Trockenextrakt aus Filipendula ulmaria (Extrakt gemäß Beispiel 1) wird mit Hilfsstoffen gemischt und zu Tabletten verpresst (Tablettenkern = Position 1 - 6). Die Tabletten werden mit einem Überzug aus Hydroxypropylmethylcellulose versehen (Position 7 -10).

| | *Bestandteil* | *mg*/*Tablette* |
|---|---|---|
| 1 | Trockenextrakt aus Filipendula ulmaria gemäß Beispiel 1 | 100.0 |
| 2 | Mikrokristalline Cellulose | 117.0 |
| 3 | Laktose Monohydrat | 58.0 |
| 4 | Croscarmellose | 15.0 |
| 5 | Hochdisperses Siliciumdioxid | 3.0 |
| 6 | Magnesiumstearat | 6.0 |
| 7 | Hydroxypropylmethylcellulose | 15.0 |
| 8 | Polyethylenglycol | 3.0 |
| 9 | Talkum | 1.0 |
| 10 | Titandioxid | 2.0 |

## Patentansprüche

1. Extrakt aus Filipendula zur Verwendung zur Behandlung oder Prophylaxe von chronischen Schmerzzuständen ohne erkennbare organische Ursachen, wobei der Extrakt durch Extraktion mit 50-70 Gew.-% Ethanol erhalten wird, wobei der Extrakt oral oder parenteral verabreicht wird und wobei die chronischen Schmerzen durch somatoforme Störungen verursacht sind ausgewählt aus Psychalgie, Kopfschmerz, Rückenschmerz und chronischer Schmerzstörung mit somatischen und psychischen Faktoren oder ausgewählt sind aus Vulvodynie oder aus Abdomen-, Schulter-, Rücken-, Lenden- oder Wirbelsäulenschmerzen nichtrheumatischen Ursprungs, oder wobei die chronischen Schmerzen durch Reizdarmsyndrom oder durch prämenstruelle Beschwerden induziert sind, oder wobei die chronischen Schmerzen durch Parästhesie der Haut induziert sind, oder wobei die chronischen Schmerzen durch Cluster-Kopfschmerz, chronische paroxysmale Hemikranie, vasomotorischen Kopfschmerz, Spannungskopfschmerz oder chronischen posttraumatischen Kopfschmerz induziert sind.

2. Extrakt aus Filipendula zur Verwendung nach Anspruch 1, wobei die Filipendulaart zur Herstellung eines Extraktes ausgewählt ist aus der Gruppe umfassend F. ulmaria, F. angustiloba, F. digitata, F. formosa, F. glaberrima, F. kamtschatica, F. kiraishiensis, F. multijuga, F. occidentalis, F. palmata, F. purpurea, F. rufinervis, F. rubra, F. vestita und F. vulgaris (syn. F. hexapetala).

3. Extrakt aus Filipendula zur Verwendung nach Anspruch 2, wobei als Filipendulaart zur Herstellung des Extraktes Filipendula ulmaria eingesetzt wird.

4. Extrakt aus Filipendula zur Verwendung nach einem der Ansprüche 1 bis 3, wobei die oberirdischen Teile (Kraut) von Filipendula eingesetzt werden.

5. Arzneimittel oder Lebensmittel, **gekennzeichnet durch** einen Gehalt an Extrakten aus Filipendula und ggf. pharmazeutisch bzw. lebensmittelrechtlich akzeptablen Hilfsstoffen, zur Verwendung in der Behandlung oder Prophylaxe von chronischen Schmerzzuständen ohne erkennbare organische Ursachen, nach einem der Ansprüche 1 bis 4.

## Claims

1. Extract from Filipendula for use in the treatment or prophylaxis of chronic pain without identifiable organic causes, wherein the extract is obtained by extraction with 50-70 wt.% ethanol, wherein the extract is administered orally or parenterally and wherein the chronic pain is caused by somatoform disorders selected from psychalgia, headache, back pain and chronic pain disorder with somatic and psychological factors or selected from vulvodynia or abdomen, shoulder, back, lumber or spinal pain which is not of rheumatic origin, or wherein the chronic pain is induced by irritable bowel syndrome or by premenstrual complaints, or wherein the chronic pain is induced by paresthesia of the skin, or wherein the chronic pain is induced by cluster headache, chronic paroxysmal hemicrania, vasomotoric headache, tension headache or chronic posttraumatic headache.

2. Extract from Filipendula for use according to claim 1, wherein the Filipendula species for production of an extract is selected from the group comprising F. ulmaria, F. angustiloba, F. digitata, F. formosa, F. glaberrima, F. kamtschatica, F. kiraishiensis, F. multijuga, F. occidentalis, F. palmata, F. purpurea, F. rufinervis, F. rubra, F. vestita and F. vulgaris (syn. F. hexapetala).

3. Extract from Filipendula for use according to claim 2, wherein Filipendula ulmaria is used as the Filipendula species for production of the extract.

4. Extract from Filipendula for use according to any one of claims 1 to 3, wherein the above-ground parts (foliage) of Filipendula are used.

5. Drug or food product, **characterized by** a content of extracts from Filipendula and optionally excipients that are pharmaceutically acceptable and acceptable according to food law for use in the treatment or prophylaxis of chronic pain without identifiable organic causes according to any one of claims 1 to 4.

## Revendications

1. Extrait de Filipendula pour l'utilisation dans le traitement ou la prophylaxie d'états douloureux chroniques sans causes organiques identifiables, l'extrait étant obtenu par l'extraction en utilisant de 50 à 70% en poids d'éthanol, l'extrait étant administré par voie orale ou parentérale, et dans laquelle les douleurs chroniques sont causées par des troubles somatoformes, choisis parmi la psychalgie, la céphalée, la notalgie et le trouble douloureux avec des facteurs somatiques et psychique ou choisis parmi la vulvodynie ou parmi les douleurs de l'abdomen, de l'épaule, du dos, des lombes ou de la colonne vertébrale qui ne sont pas d'origine rhumatismale, ou dans laquelle les douleurs chroniques sont induites par le syndrome du côlon irritable ou par les symptômes prémenstruels, ou dans laquelle les douleurs chroniques sont induites par la paresthésie de la peau, ou dans laquelle les douleurs chroniques sont induites par les algies vasculaires de la face, l'hémicrânie paroxysmale chronique, les maux de tête vasomoteurs, les céphalées de tension ou la céphalée post-traumatique chronique.

2. Extrait de Filipendula pour l'utilisation selon la revendication 1, l'espèce de Filipendula pour la production d'extrait étant choisie parmi le groupe comprenant F. ulmaria, F. angustiloba, F. digitata, F. formosa, F. glaberrima, F. kamtschatica, F. kiraishiensis, F. multijuga, F. occidentalis, F. palmata, F. purpurea, F. rufinervis, F. rubra, F. vestita et F. vulgaris (syn. F. hexapetala).

3. Extrait de Filipendula pour l'utilisation selon la revendication 2, Filipendula ulmaria étant utilisé en tant que l'espèce de Filipendula pour la fabrication de l'extrait.

4. Extrait de Filipendula pour l'utilisation selon l'une quelconque des revendications 1 à 3, dans lequel les parties aériennes (herbe) de Filipendula sont utilisées.

5. Médicament ou aliment, **caractérisé par** une teneur en extraits de Filipendula et éventuellement des excipients pharmaceutiquement acceptables ou approuvés par la législation alimentaire pour l'utilisation dans le traitement ou la prophylaxie d'états douloureux chroniques sans causes organiques identifiables selon l'une quelconque des revendications 1 à 4.
